# FASCICULE DE BREVET EUROPEEN

(11) **EP 2 496 554 B1**
(45) Date de publication et mention de la délivrance du brevet: **04.03.2015**
(21) Numéro de dépôt: 10776639.6
(22) Date de dépôt: 02.11.2010
(51) Int. Cl.: C07C 407/00, C07C 409/02, C07C 409/14, B01J 19/02

(54) **PROCEDE DE PREPARATION DE COMPOSES HYDROPEROXYDE D'ALKYLE**
VERFAHREN ZUR HERSTELLUNG VON ALKYLHYDROPEROXIDVERBINDUNGEN
METHOD FOR PREPARING ALKYL HYDROPEROXIDE COMPOUNDS

(30) Priorité: 05.11.2009 FR 0957834
(43) Date de publication de la demande: 12.09.2012
(73) Titulaire: Rhodia Operations, 93306 Aubervilliers (FR)
(72) Inventeur: BELLENGER, Fabien, Shanghai 200030 (CN); DIGUET, Laurent, F-69800 Saint Priest (FR); STREIFF, Stéphane, F-69006 Lyon (FR)
(74) Mandataire: Chatelan, Florence Anne
(86) Numéro de dépôt international: PCT/EP2010/066616
(87) Numéro de publication internationale: WO 2011/054809

(56) Documents cités:
- US-A- 4 326 084
- GALEN, J.SUPPES; MCHUGH, M.A.: "Solvent and catalytic metal effects on the decomposition of cumene hydroperoxide", INDUSTRIAL & ENGINEERING CHEMISTRY RESEARCH, vol. 28, no. 8, 1 janvier 1989 (1989-01-01), pages 1146-1152, XP002593917,
- DATABASE WPI Week 198849 Thomson Scientific, London, GB; AN 1988-350438 XP002623570, & JP 63 262481 A (TECHN OF SURFACE KK) 28 octobre 1988 (1988-10-28)
- Yoshio Kamiya: "ORGANIC OXIDATION REACTION - theory and application of automatic oxidation -", 5 August 1973 (1973-08-05), Gihodo Co., Tokyo pages 187-190,
- Falbe, J. and Regitz, M.: "Römpp Lexikon Chemie", 31 December 1999 (1999-12-31), Georg Thieme Verlag, Stuttgart vol. 10, page 4417,
- 'Polymer Handbook', 31 Décembre 1999, WILEY-INTERSCIENCE, NEW JERSEY Bd. 'Physical Constants of Fluoropolymers', page 31,35,41,43
- 'High Performance Fluoropolymer Coatings & Linings', [en ligne] 31 Décembre 2006, Extrait de l'Internet: <URL:http://www.solvaysites.com/sites/solva y/plastics/EN/Solvay%20Plastics%20Literatur e/BR_Coatings_Linings_EN.pdf>

## Description

La présente invention concerne un procédé de fabrication de composés hydroperoxyde d'alkyle, plus particulièrement la préparation d'hydroperoxyde de cyclohexyle.

Elle se rapporte plus particulièrement à la préparation d'hydroperoxyde de cyclohexyle par oxydation par l'oxygène de cyclohexane dans un réacteur multi-étages ou des réacteurs montés en série.

L'hydroperoxyde de cyclohexyle est un produit intermédiaire obtenu dans la synthèse de l'acide adipique par oxydation du cyclohexane par l'oxygène.

Ainsi, dans un procédé exploité industriellement, le cyclohexane liquide est oxydé par réaction avec l'oxygène gaz dans un réacteur multi-étages, à haute température, en hydroperoxyde de cyclohexane. L'hydroperoxyde, après séparation du cyclohexane n'ayant pas réagi, est décomposé en cyclohexanol et cyclohexanone, en présence d'un catalyseur. Le mélange cyclohexanol/cyclohexanone appelé généralement "olone" est ensuite oxydé en acide adipique et autres diacides par l'acide nitrique. L'acide adipique est extrait et purifié, avantageusement par cristallisation.

Dans ce procédé, il est important d'obtenir un rendement le plus élevé possible en hydroperoxyde de cyclohexyle. Ce rendement est fonction des conditions de mise en oeuvre de la réaction, telles que la température, la concentration en oxygène, par exemple.

Toutefois, pour éviter de dégrader ce rendement, il est nécessaire d'éviter la décomposition de l'hydroperoxyde de cyclohexyle. En effet, ce composé peut se décomposer rapidement si le milieu contient des espèces métalliques qui ont une activité catalytique élevée. De telles espèces métalliques peuvent souvent provenir de la corrosion des surfaces métalliques du réacteur, des éléments internes contenus dans le réacteur ainsi que des appareils ou dispositifs annexes aux réacteurs, dans lesquels circule le milieu réactionnel.

Pour éviter et limiter ce phénomène, il a été proposé depuis de nombreuses années un procédé de pyrophosphatation des surfaces métalliques tel que cela est décrit dans le brevet US3510526. Pour obtenir cette pyrophosphatation, le réacteur et ses éléments internes sont soumis à un traitement avec un pyrophosphate avant le début de la réaction d'oxydation. Il est nécessaire de répéter périodiquement ce traitement ce qui entraine une baisse du taux de fonctionnement de l'installation et donc affecte l'économie du procédé.

Par ailleurs, comme les conditions de fonctionnement du réacteur sont très sévères, températures et pression élevées, présence de composés à fort pouvoir oxydant (oxygène, peroxydes), la protection des surfaces métalliques par des matériaux protecteurs usuels utilisés dans les réacteurs chimiques ne peut être mise en oeuvre de manière efficace, la pyrophosphatation étant pratiquement la seule solution utilisée actuellement. US4326084 décrit un procédé de préparation d'un mélange contenant du cyclohexanol et de la cyclohexanone à partir de cyclohexane. A titre d'exemple, les parois internes du réacteur et la pale d'agitation sont recouvertes de résine fluorocarbonée Teflon®.

Un des buts de la présente invention est de proposer une solution permettant de réaliser une protection des surfaces métalliques efficaces dans les conditions de fonctionnement du réacteur et ne requérant pas un remplacement fréquent ni des arrêts périodiques de l'installation.

A cet effet, l'invention propose un procédé de préparation de composés hydroperoxyde d'alkyle, plus particulièrement d'hydroperoxyde de cyclohexyle, comprenant la mise en contact d'un hydrocarbure liquide, plus particulièrement le cyclohexane, dans un réacteur à une température et pression élevées avec l'oxygène. Selon l'invention les surfaces du réacteur en contact avec le milieu contenant l'hydroperoxyde comprennent une couche protectrice réalisée en un polymère perfluoroalcoxy, généralement désigné par l'appellation "polymère PFA".

Selon une autre caractéristique de l'invention, le réacteur peut comprendre des éléments internes tels que plateaux, agitateurs, sondes etc. Les surfaces de ces éléments en contact avec le milieu contenant l'hydroperoxyde d'alkyle sont avantageusement protégées par une couche protectrice réalisée en "polymère PFA". De même, les dispositifs et installations annexes au réacteur, tels que pompes, conduites de circulation, appareil de flashage du cyclohexane peuvent être avantageusement protégés par un revêtement conforme à l'invention, c'est-à-dire un revêtement en polymère PFA. De même, pour éviter la décomposition de l'hydroperoxyde d'alkyle pendant une phase de stockage, le dispositif de stockage est avantageusement protégé par une couche protectrice réalisée en polymère PFA.

Il existe un grand nombre de polymères fluorés. Les polymères PFA sont une famille particulière de polymères fluorés. Un des plus connu parmi les polymères fluorés est le PTFE (polyterafluoroéthylène) commercialisé sous la marque TEFLON^{®}.

Les polymères PFA ou perfluoroalcoxy sont différents des polymères PTFE notamment par leur caractère fusible, c'est-à-dire qu'ils peuvent être fondus et donc mis en forme par les procédés classiques utilisés avec les polymères thermoplastiques tels que le moulage par injection, l'extrusion. Le PTFE ne peut donc pas être mis en forme facilement dans des installations, par exemple pour recouvrir des parois internes des réacteurs. En outre, le PTFE présente une porosité élevée ce qui engendre des phénomènes d'absorption et de désorption de certains produits chimiques en cours de réaction comme les alcools, cétones et les acides produits par l'oxydation de l'hydrocarbure. Ainsi, l'utilisation du PTFE présente un grand nombre d'inconvénients particulièrement gênants dans le cadre du procédé de l'invention.

Ces polymères ont été inventés par la société DUPONT de Nemours et sont notamment commercialisés sous le nom commercial TEFLON-PFA. D'autres sociétés commercialisent des polymères équivalents, telle que, par-exemple, la société DAIKIN sous le nom commercial NCOFLON-PFA, ou SOLVAY sous le nom HYFLON-PFA ou encore la société GORE, et plus généralement les polymères PFA résistant thermiquement et chimiquement.

Les polymères PFA peuvent également comprendre des charges minérales ou organiques pour améliorer leurs propriétés mécaniques. Toutefois, dans le cadre de l'invention ces charges ne doivent pas comprendre d'éléments métalliques.

A titre d'exemple de polymère PFA convenable pour l'invention on peut citer les différents grades commercialisés par la société DU PONT DE NEMOURS sous la dénomination commerciale PFA Ruby Red^{®} ou par la société GORE sous la dénomination commerciale FLUOROSHIELD^{®}.

La couche protectrice en polymères PFA peut être réalisée selon les méthodes habituelles de dépôt de polymère sur une surface ou par l'application de plaques ou feuilles en PFA sur la surface à protéger.

Avantageusement, la couche protectrice en PFA a une épaisseur comprise entre 500 µm et 1 mm.

Ainsi, à titre d'illustration, on peut citer comme procédés de dépôt de polymère, la technologie de poudrage électrostatique qui consiste à appliquer plusieurs couches de polymères et de passer l'appareil protégé dans un four pour faire ramollir ou fondre les grains de plastiques pour obtenir un film continu. Ce procédé d'application comprend une étape de préparation des surfaces à revêtir pour les dégraisser, nettoyer et supprimer les défauts de surface notamment au niveau des soudures. A titre d'exemple, on peut citer le procédé de sablage qui permet d'obtenir un état de surface convenable dont la qualité est indiquée par le degré SA défini dans la norme SIS 05 59 00. Après ce nettoyage, une couche de primaire d'accrochage est avantageusement appliquée sur la surface. Cette couche de primaire peut avantageusement comprendre un polymère PFA. Le revêtement protecteur est ensuite réalisé par dépôt d'une ou plusieurs couches successives de polymères PFA, avantageusement par projection électrostatique d'une poudre PFA en suspension ou non dans un liquide.

D'autres techniques de réalisation du revêtement peuvent être utilisées telles que celle proposée par la société GORE. Ainsi, dans le système commercialisé par cette société sous le nom commercial FLUOROSHIELD^{®,} le procédé consiste à fixer par exemple par soudage, une grille en matériau de type INCONEL^{®} sur le support à protéger et à appliquer une couche de polymère de PFA sur cette grille. Ainsi, l'accrochage de la protection en polymère PFA est renforcé.

En outre, cette protection est avantageusement utilisée pour protéger des surfaces métalliques, notamment des surfaces en acier inoxydable, mais elle peut être utilisée pour protéger tout type de surface métallique ou non.

Dans un mode de réalisation de l'invention de préparation d'hydroperoxyde d'alkyle décrit à titre d'exemple, le procédé consiste à introduire l'hydrocarbure tel que le cyclohexane à la partie inférieure d'un réacteur tubulaire, simultanément à l'introduction d'oxygène ou d'un gaz contenant de l'oxygène en continu. Toutefois, l'invention s'applique également aux procédés mettant en oeuvre d'autres types de réacteurs, notamment avec introduction étagée de l'oxygène ou introduction de l'hydrocarbure en tête du réacteur.

La pression de la réaction est comprise entre 10 et 30 bars, la température est comprise entre 130°C et 200°C. L'oxygène est introduit en continu dans le ciel du réacteur avec un débit compris entre 10 et 30 Nl/h. Pour des raisons de sécurité, les conditions de la réaction, notamment le temps de séjour dans le réacteur est contrôlé pour obtenir, avantageusement, dans le ciel du réacteur une phase gaz contenant de l'oxygène à une concentration inférieure à une valeur critique, de l'ordre de 5% en volume, pour limiter les risques d'auto-explosion et d'auto-inflammation avec les vapeurs d'hydrocarbure. Toutefois, cette caractéristique n'est pas obligatoire.

Le procédé de l'invention permet de limiter la décomposition de l'hydroperoxyde formé par la réaction d'oxydation et donc améliorer le rendement de la réaction, en limitant le contact entre le milieu réactionnel et les surfaces métalliques.

Selon l'invention, il est également possible de réaliser sur la couche protectrice en polymère PFA, une pyrophosphatation.

En outre, la couche protectrice en polymère PFA a une durée de vie très longue et donc permet d'améliorer l'économie du procédé en diminuant la fréquence des arrêts nécessaires pour renouveler ou réparer la protection.

Par ailleurs, la couche protectrice en polymère PFA présente l'avantage de ne pas absorber ni relarguer les composés chimiques produits en cours de réaction tels que le cyclohexanol, la cyclohexanone et les sous produits formés au cours de la réaction comme par exemples les acides, ce qui limite les arrêts pour nettoyage du réacteur.

Elle constitue aussi une barrière efficace à l'interaction entre les parois métalliques et le milieu réactionnel, ce qui permet de limiter la décomposition de l'hydroperoxyde et donc d'améliorer le rendement de la réaction.

De plus, la couche protectrice en polymère PFA permet de travailler à haute température, elle présente une meilleure résistance au fluage, c'est-à-dire à la déformation sous une contrainte permanente. A titre d'exemple, sous une charge de 8,8 MPa (Megapascal) à 150°C pendant 100 heures, et après 24 heures de relaxation, le PFA a un taux de déformation rémanent de 10% alors que celui-ci est de 34% pour le PTFE (test réalisé selon la norme ASTM D621).

D'autres avantages, détails de l'invention apparaîtront plus clairement au vu des exemples donnés ci-dessous à titre d'illustration.

### EXEMPLES

Du cyclohexane est chargé dans un réacteur équipé d'une turbine auto-aspirante et d'un condenseur. Le réacteur en acier inoxydable est soit non revêtu soit pyrophosphaté soit revêtu d'une couche de PFA. Le milieu réactionnel est chauffé à 165°C. Quand la température est atteinte, de l'oxygène à 43% en volume dans de l'azote est injecté à un débit de 30 Nl/h sous une pression de 30 bars. Des échantillons sont prélevés puis analysés en chromatographie en phase gazeuse pour suivre la formation de peroxyde de cyclohexyle au cours du temps. Aucune différence de la sélectivité en HPOCH en fonction du taux de transformation n'est observée entre un réacteur pyrophosphaté et un réacteur revêtu d'une couche de PFA par contre une grande différence existe en comparaison d'un réacteur non revêtu.

### - Protocole de pyrophosphatation du réacteur :

Une solution aqueuse de pyrophosphate de sodium 5% massique est introduite dans le réacteur en acier inoxydable. Après 45 minutes d'agitation à température ambiante le réacteur est vidé puis laissé sécher une nuit à température ambiante.

### - Protocole de réalisation d'un revêtement PFA conforme à l'invention, de la surface interne du réacteur c :

### Préparation du support :

La surface à revêtir subit des traitements habituels de dégraissage, nettoyage et sablage. Les soudures et défauts de surfaces sont arasés. Ce traitement peut être localisé ou général à toute la pièce. L'état final des surfaces de pièces à revêtir est mesuré et qualifié par l'indice SA défini dans la norme SIS 05 59 00. Cet indice est de 2.5.

### Application du revêtement :

L'application se fait par pulvérisation d'une poudre PFA commercialisée par la société DU PONT en suspension dans de l'eau. Cette application comprend la projection électrostatique d'une couche de primaire d'accrochage à base de polymère PFA. Le réacteur est chauffé à une température voisine de 400°C pour permettre la cuisson de la couche de primaire. La température de cuisson est généralement fournie par le fabricant de polymère PFA commercialisé par la société DU PONT sous la dénomination commerciale RUBY RED^{®} et dépend de la nature de ce polymère. Une première couche de polymère PFA est ensuite appliquée sur la couche de primaire selon la même technique, puis le réacteur est à nouveau porté à 400°C pour cuire cette couche. Cette opération est répétée autant de fois que nécessaire pour obtenir l'épaisseur désirée de la couche protectrice. Par exemple, selon les systèmes, le poudrage électrostatique est utilisé. La qualité et l'épaisseur de la couche protectrice sont contrôlées notamment l'homogénéité et l'adhérence de la couche protectrice.

Des essais de production d'hydroperoxyde de cyclohexyle ont été réalisés dans un réacteur non protégé, un réacteur protégé par pyrophosphatation et un réacteur protégé, conformément à l'invention, par un revêtement PFA, avec des conditions opératoires identiques décrites ci-dessus.

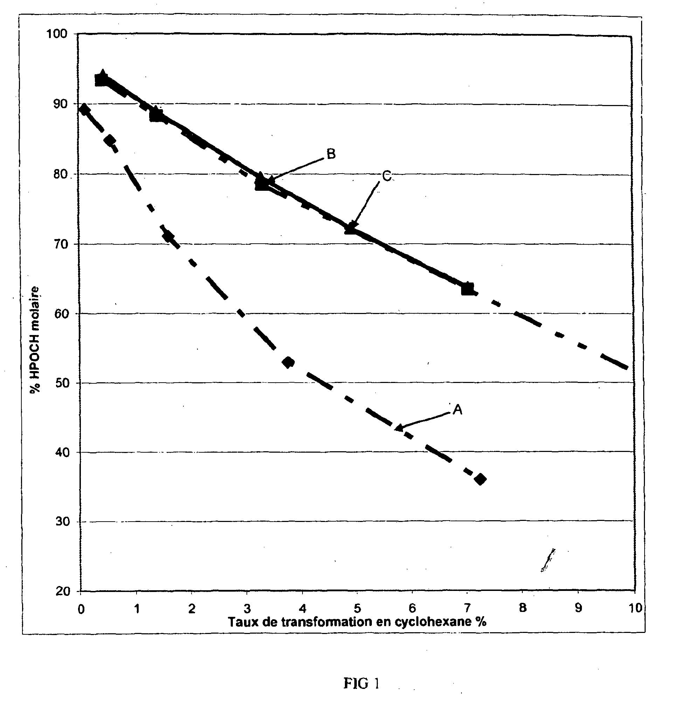

Le pourcentage molaire d'hydroperoxyde de cyclohexyle (HPOCH) produit a été mesuré pour différents taux de transformation de cyclohexane. Les résultats obtenus sont rassemblés dans la figure 1 qui représente la variation du taux molaire de HPOCH produit en fonction du taux de transformation de cyclohexane pour les trois réacteurs utilisés :
- Courbe A : essai dans réacteur en inoxydable non protégé
- Courbe B : essai dans réacteur en inoxydable protégé par pyrophosphatation
- Courbe C : essai dans réacteur en inoxydable protégé par revêtement PFA conforme à l'invention

Ces résultats montrent que le revêtement conforme à l'invention assure une protection équivalente à celle obtenue avec le procédé de pyrophosphatation.

## Revendications

1. Procédé de préparation d'hydroperoxyde d'alkyle comprenant la mise en contact d'un hydrocarbure liquide dans un réacteur à une température élevée avec l'oxygène, **caractérisé en ce que** les surfaces du réacteur en contact avec le milieu contenant l'hydroperoxyde comprennent une couche protectrice réalisée en un polymère perfluoroalcoxy (PFA).

2. Procédé selon la revendication 1, **caractérisé en ce que** des éléments internes sont disposés dans le réacteur, la surface desdits éléments en contact avec le milieu contenant l'hydroperoxyde d'alkyle comprend une couche protectrice réalisée en un polymère perfluoroalcoxy (PFA).

3. Procédé selon les revendications 1 ou 2, **caractérisé en ce que** la couche protectrice a une épaisseur comprise entre 500 µm et 1 mm.

4. Procédé selon la revendication 1 à 3, **caractérisé en ce que** la couche protectrice est obtenue par dépôt du polymère PFA.

5. Procédé selon la revendication 1 à 3, **caractérisé en ce que** la couche protectrice est formée par un film de PFA disposé sur les surfaces à protéger.

6. Procédé selon l'une des revendications 1 à 3 **caractérisé en ce que** la couche protectrice est réalisée par disposition et juxtaposition de plaques en PFA sur les surfaces à protéger.

7. Procédé selon l'une des revendications précédentes, **caractérisé en ce que** l'hydroperoxyde d'alkyle est l'hydroperoxyde de cyclohexyle, l'hydrocarbure étant le cyclohexane.

## Patentansprüche

1. Verfahren zur Herstellung eines Alkylhydroperoxids, bei dem man einen flüssigen Kohlenwasserstoff in einem Reaktor bei einer hohen Temperatur mit Sauerstoff in Kontakt bringt, **dadurch gekennzeichnet, dass** die Oberflächen des Reaktors in Kontakt mit dem das Hydroperoxid enthaltenden Medium eine Schutzschicht aus einem Perfluoralkoxypolymer (PFA-Polymer) aufweisen.

2. Verfahren nach Anspruch 1, **dadurch gekennzeichnet, dass** in dem Reaktor Einbauten angeordnet sind und die Oberfläche der Einbauten in Kontakt mit dem das Hydroperoxid enthaltenden Medium eine Schutzschicht aus einem Perfluoralkoxypolymer (PFA-Polymer) aufweisen.

3. Verfahren nach Anspruch 1 oder 2, **dadurch gekennzeichnet, dass** die Schutzschicht eine Dicke zwischen 500 µm und 1 mm aufweist.

4. Verfahren nach Anspruch 1 bis 3, **dadurch gekennzeichnet, dass** die Schutzschicht durch Abscheidung von PFA-Polymer erhalten wird.

5. Verfahren nach Anspruch 1 bis 3, **dadurch gekennzeichnet, dass** die Schutzschicht durch einen auf den zu schützenden Oberflächen angeordneten PFA-Film gebildet wird.

6. Verfahren nach einem der Ansprüche 1 bis 3, **dadurch gekennzeichnet, dass** die Schutzschicht durch Anordnen und Nebeneinanderstellen von PFA-Platten auf den zu schützenden Oberflächen hergestellt wird.

7. Verfahren nach einem der vorhergehenden Ansprüche, **dadurch gekennzeichnet, dass** es sich bei dem Alkylhydroperoxid um Cyclohexylperoxid handelt, wobei es sich bei dem Kohlenwasserstoff um Cyclohexan handelt.

## Claims

1. Process for preparing an alkyl hydroperoxide, comprising the placing of a liquid hydrocarbon in a reactor at a high temperature in contact with oxygen, **characterized in that** the surfaces of the reactor in contact with the medium containing the hydroperoxide comprise a protective coat made of a perfluoroalkoxy (PFA) polymer.

2. Process according to Claim 1, **characterized in that** internal components are arranged in the reactor, and the surface of the said components in contact with the medium containing the alkyl hydroperoxide comprises a protective coat made of a perfluoroalkoxy (PFA) polymer.

3. Process according to Claim 1 or 2, **characterized in that** the protective coat has a thickness of between 500 µm and 1 mm.

4. Process according to Claims 1 to 3, **characterized in that** the protective coat is obtained by depositing PFA polymer.

5. Process according to Claims 1 to 3, **characterized in that** the protective coat is formed by a PFA film deposited onto the surfaces to be protected.

6. Process according to one of Claims 1 to 3, **characterized in that** the protective coat is produced by arrangement and juxtaposition of PFA plates on the surfaces to be protected.

7. Process according to one of the preceding claims, **characterized in that** the alkyl hydroperoxide is cyclohexyl hydroperoxide, the hydrocarbon being cyclohexane.
